# EUROPEAN PATENT APPLICATION

(11) **EP 4 613 877 A1**
(43) Date of publication of application: **10.09.2025**
(21) Application number: 23886355.9
(22) Date of filing: 03.11.2023
(51) Int. Cl.: C12Q 1/6881, C12N 5/0797

(54) **METHOD FOR DIFFERENTIATING NEURAL CREST STEM CELLS COMPRISING AXIAL SPECIFICATION INFORMATION**

(30) Priority: 03.11.2022 KR 20220145111
(71) Applicant: Bless Biotherapeutics Inc., Gyeonggi-do 18455 (KR)
(72) Inventor: KIM, Yong Jun, Seoul 02423 (KR)
(74) Representative: Schön, Christoph
(86) International application number: PCT/KR2023/017488
(87) International publication number: WO 2024/096661

(57) **Abstract**

The present disclosure relates to a method of selectively differentiating and isolating, from pluripotent stem cells, neural crest cells having axial specification information in the body. According to the method of the present disclosure, initial neural crest cells, which correspond to an embryo neurulation stage and have not yet formed axial information in the body, can be produced, neural crest cells having specific axial information in the human body can be produced, and the cells can be specifically isolated and cultured in vitro such that various types of neural crest cells can be obtained all at once, and biological processes of neural crest cells for retaining axial information are researched to identify developmental processes of tissues and organs, and thus various cell therapeutic agents optimized for the characteristics of locations in the human body can be developed.

## Description

### Technical Field

The present disclosure relates to a method of selectively differentiating and isolating, from pluripotent stem cells, neural crest cells having axial specification information in the human body.

### Background Art

Cells of the nervous system can be broadly divided into two types: cells of the central nervous system and motor nerve cells constituting the brain and spinal cord; and cells of the peripheral nervous system constituting sensory nerves, autonomic nerves, and the like. Neurons, astrocytes, and oligodendrocytes, which constitute the central nervous system (brain and spinal cord) and motor nerves, may be generated by differentiating neural stem cells or neural progenitor cells (NPCs) differentiated from pluripotent stem cells, whereas peripheral neurons (autonomic nerves and sensory nerves) and Schwann cells, which constitute the peripheral nervous system, are derived from neural crest stem cells (NCSCs) differentiated from pluripotent stem cells. Therefore, cells of the central nervous system and cells of the peripheral nervous system are generated from pluripotent stem cells via NPCs and NCSCs, respectively, according to different differentiation pathways, and these different pathways are known to be dependent on the surrounding environment and intracellular signaling systems.

NCSCs existing in the human body during embryogenesis or fetal development are widely located along the rostral-caudal axis, from the head and neck to the coccyx, and NCSCs, which are subdivided into cranial NCSCs, trunk NCSCs, cardiac NCSCs, and sacral NCSCs depending on the location of the rostral-caudal axis in the human body, may have, depending on the type, different cells, tissues, and organs as differentiation targets. During embryogenesis, initial NCSCs do not have information of the rostral-caudal axis, but at the time of neurulation together with embryo elongation, the neural crest naturally acquires location information of the rostral-caudal axis localization as it moves along the elongating rostral-caudal axis, thereby showing distinction among cranial NCSCs, trunk NCSCs, cardiac NCSCs, and sacral NCSCs. The NCSCs migrate from the posterior part of the neural folds during the migratory stage of development, and migrate to be distributed in a wide range of tissues and organs in the body. After the migratory stage, the NCSCs are known to differentiate into neurons of the peripheral nervous system, glial cells, skin melanocytes, endocrine cells, and various mesenchymal cells, which are then distributed to neural or non-neural tissues and organs in adults (Dev Dyn 2007;236:3242).

Recently, it has been discovered that NCSC-like cells exist even after birth, in the peripheral nerves, dorsal root ganglia, guts, hair follicles, dermal papillae, cornea, and dental pulps. Among these, adult human NCSC-like cells were isolated only from hair follicles, dermal papillae, and dental pulps (J Cell Biochem 2009;107:1046). Previously, NCSCs isolated from dermal papillae or hair follicles in human adults have been reported. However, since dermal papillae or hair follicles include many epidermal cell-derived appendages, neurosphere-forming cells from responsible tissues are likely to be mixed with heterogeneous cells derived from skin appendages in addition to the NCSCs, causing a problem in the application as cell therapy agents (PNAS 2005;102:5530). In addition, although previous research results have proven that NCSCs or NCSC-like cells exist in adult humans, the previous technology requires long-term cell culture of 3 months to isolate and culture NCSCs. In addition, due to such prolonged cell culture, it is not possible to exclude the possibility that various cellular transformations such as transformation and dedifferentiation may have occurred. In addition, considering the characteristics of NCSCs that differentiate into various cells located and functioning in each organ or tissue in the human body, adult NCSCs or NCSC-like cells cultured using conventional techniques have likely already undergone significant differentiation and lost their initial pluripotency. In addition, conventional techniques have used specific markers to isolate NCSCs, but to date, there is no specific marker that can specifically represent according to the developmental stage of NCSCs.

### Description of Embodiments

### Technical Problem

An object of the present disclosure is to provide a method of producing initial neural crest stem cells.

In addition, an object of the present disclosure is to provide a method of producing neural crest stem cells.

In addition, an object of the present disclosure is to provide a composition for detecting initial neural crest stem cells.

In addition, an object of the present disclosure is to provide a method of detecting initial neural crest stem cells.

In addition, an object of the present disclosure is to provide use for detecting initial neural crest stem cells.

In addition, an object of the present disclosure is to provide use for preparing a composition for detecting initial neural crest stem cells.

In addition, an object of the present disclosure is to provide a composition for detecting neural crest stem cells.

In addition, an object of the present disclosure is to provide a method of detecting neural crest stem cells.

In addition, an object of the present disclosure is to provide use for detecting neural crest stem cells.

Furthermore, an object of the present disclosure is to provide use for preparing a composition for detecting neural crest stem cells in preparation.

### Solution to Problem

To achieve these objects, the present disclosure provides a composition for detecting initial neural crest stem cells, including an agent for measuring an expression level of the p75 NGFR gene or the HNK1 gene.

In addition, the present disclosure is to provide a method of detecting initial neural crest stem cells, the method including measuring an expression level of the p75 NGFR gene or the HNK1 gene in an isolated sample.

In an embodiment, the sample may be any material, biological fluid, tissue, or cell derived from a subject.

In an embodiment, the method of detecting initial neural crest stem cells may further include determining cells as initial neural crest stem cell if the expression level of the p75 NGFR or HNK1 gene is increased or positive compared to a control group.

In addition, the present disclosure provides use of an agent for measuring an expression level of the p75 NGFR or HNK1 gene for detecting initial neural crest stem cells.

In addition, the present disclosure provides use of an agent for measuring an expression level of the p75 NGFR or HNK1 gene for preparing a composition for detecting initial neural crest stem cells.

In addition, the present disclosure provides use of p75 NGFR or HNK1 for detecting initial neural crest stem cells.

In addition, the present disclosure provides a composition for detecting neural crest stem cells having axial specification information, the composition including an agent for measuring an expression level of the ETS1 gene or the ZIC1 gene.

In addition, the present disclosure provides a method of detecting neural crest stem cells having axial specification information, the method including measuring an expression level of the ETS1 gene or the ZIC1 gene in an isolated sample.

In an embodiment, the sample may be any material, biological fluid, tissue, or cell derived from a subject.

In an embodiment, the method of detecting neural crest stem cells having axial specification information may further include determining cells as neural crest stem cells having axial specification information when the expression level of the ETS1 gene or the ZIC1 gene is increased or positive compared to a control group.

In addition, the present disclosure provides use of an agent for measuring an expression level of the ETS1 gene or the ZIC1 gene for detecting neural crest stem cells having axial specification information.

In addition, the present disclosure provides use of an agent for measuring an expression level of the ETS1 gene or the ZIC1 gene for preparing a composition for detecting neural crest stem cells having axial specification information.

In addition, the present disclosure provides use of the ETS1 gene or the ZIC1 gene for detecting neural crest stem cells having axial specification information.

In addition, the present disclosure provides a method of producing initial neural crest stem cells.

Furthermore, the present disclosure provides a method of producing neural crest stem cells.

### Advantageous Effects of Disclosure

According to the methods of the present disclosure, there are effects in that initial neural crest cells, which correspond to an embryo neurulation stage and have not yet formed axial information in the human body, may be produced, neural crest cells having specific axial information in the human body may be produced, and these neural crest cells may be specifically isolated and cultured in vitro such that various types of neural crest cells may be obtained all at once, and biological processes of neural crest cells for retaining axial information are researched to identify developmental processes of tissues and organs, and thus various therapeutic agents optimized for the characteristics of locations in the human body can be developed.

### Brief Description of Drawings

FIG. 1 depicts stages of intra-embryonic cell development during the human embryonic development.
FIG. 2 shows identification of cell surface markers for isolation of initial neural crest stem cells:
   (a): a chick embryo; and
   (b): human pluripotent stem cells.
FIG. 3 shows expression of initial neural crest stem cell markers (P75 NGFR and HNK1) and an axial specification marker (HOX) in initial neural crest stem cells differentiated from pluripotent stem cells for 5 days.
FIG. 4 shows a production process of neural crest stem cells.
FIG. 5 shows a comparison of gene expression levels of initial neural crest stem cells on day 5 of differentiation with neural crest stem cells having axial specifications on day 10 of differentiation.
FIG. 6 shows expression levels of ZIC1 and ETS1 when only initial neural crest stem cells exist without treatment with an axial specification stimulating factor.
FIG. 7 analyzes expression levels of neural crest stem cells that have undergone second differentiation by adding an axial specification stimulating factor:
   (a): ETS1/ZIC1 expression ratio of a group cultured by treating a cell signaling protein without isolating neural crest stem cells;
   (b): ETS1/ZIC1 expression ratio of a group cultured by treating a cell signaling protein during a process of isolating only initial neural crest stem cells and further culturing the cells alone;
   (c): expression levels of the TWIST1 gene (marker gene of cranial neural crest stem cells) and the HOX gene in initial neural crest stem cells treated with FGF2 during a secondary differentiation stage;
   (d): gene expression levels of the ZIC1 gene and the ETS1 in single cells after treatment with FGF2 at the secondary differentiation stage; and
   (e): expression ratio of ETS1/ZIC1 in single cells after treatment with FGF2 at the secondary differentiation stage.

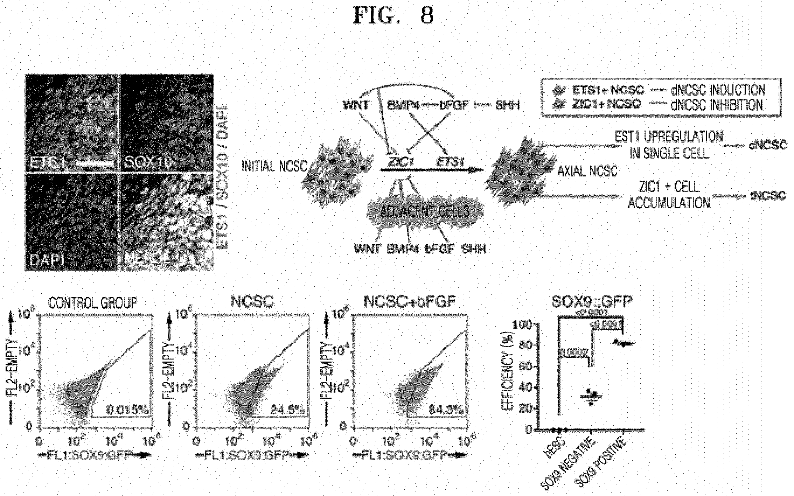

FIG. 8 analyzes a mechanism of axial specification retention of initial neural crest stem cells by a signal transduction factor.

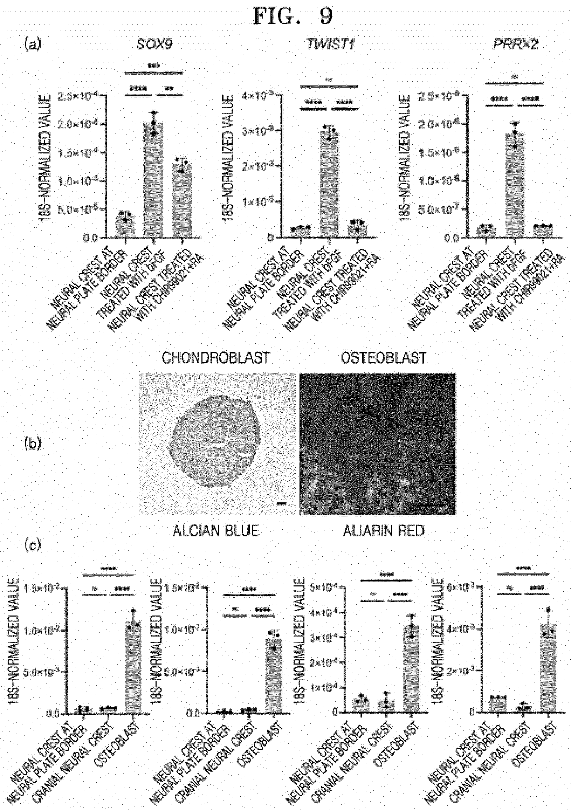

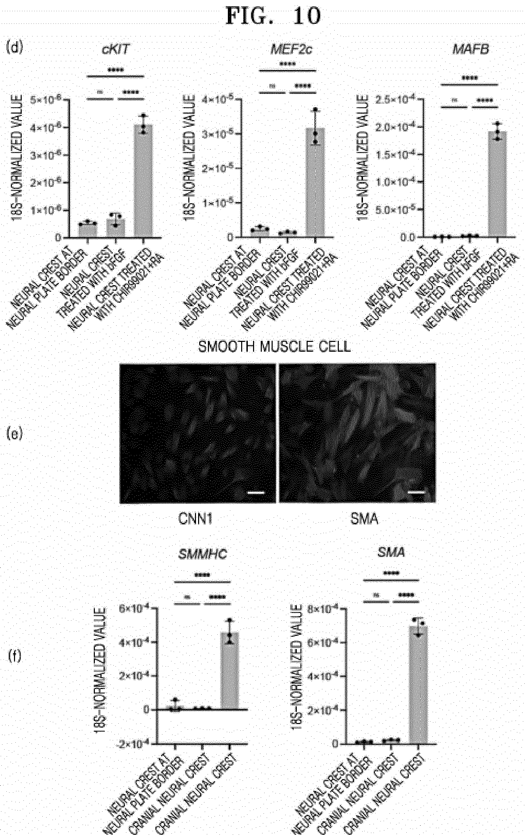

FIGS. 9 and 10 confirms simultaneous generation of multiple axially specified neural crest populations within a single batch by treating initial neural crest stem cells isolated through first differentiation with various axial specification stimulating factors:
   (a): mRNA expression level of marker gene of cranial neural crest stem cells;
   (b) and (c): validation of differentiation potential of cranial neural crest stem cells into target differentiation cells;
   (d): mRNA expression level of marker genes of cardiac neural crest stem cells; and
   (e) and (f): validation of differentiation potential of cardiac neural crest stem cells into target differentiation cells.

### Mode for Invention

Hereinafter, the present disclosure will be described in detail with reference to the attached drawings, which illustrate embodiments of the present disclosure. However, the following embodiments are presented as examples of the present disclosure, and detailed descriptions of techniques or configurations known to those skilled in the art may be omitted if it is determined that such detailed descriptions would unnecessarily obscure the gist of the present disclosure, and the present disclosure is not limited thereby. The present disclosure is capable of various modifications and applications within the scope of the following claims and equivalents interpreted therefrom.

**In** addition, the terminologies used in the present specification are intended to appropriately express preferred embodiments of the present disclosure, which may vary depending on the user, the intent of the operator, or the conventions of the field to which the present disclosure belongs. Therefore, definitions of these terminologies should be based on the context of the present specification as a whole. Throughout the specification, when a portion is said to "include" an element, it is meant to be inclusive of other components, not exclusive of other components, unless specifically noted to the contrary.

Unless otherwise defined, all technical and scientific terminologies used in the present specification have the same meaning as generally understood by those skilled in the art to which the present disclosure belongs . Any methods and materials similar or equivalent to those described herein may be used in practice to test the present disclosure, but preferred materials and methods are described herein.

In an aspect, the present disclosure relates to a composition for detecting initial neural crest stem cells, the composition including an agent for measuring an expression level of the p75 NGFR gene or the HNK1 gene.

In an aspect, the present disclosure relates to a method of detecting initial neural crest stem cells, the method including measuring an expression level of the p75 NGFR gene or the HNK1 gene in an isolated sample.

In an embodiment, the sample may be any material, a biological fluid, a tissue, or a cell derived from a subject.

In an embodiment, the method of detecting initial neural crest stem cells may further include determining cells as initial neural crest stem cells if the expression level of the p75 NGFR gene or the HNK1 gene is increased or positive compared to a control group.

In an aspect, the present disclosure relates to use of the agent for measuring the expression level of the p75 NGFR gene or the HNK1 gene, for detecting initial neural crest stem cells.

In an aspect, the present disclosure relates to use of the agent for measuring the expression level of the p75 NGFR gene or the HNK1 gene, for preparing a composition for detecting initial neural crest stem cells.

In an aspect, the present disclosure provides use of the p75 NGFR gene or the HNK1 gene, for detecting initial neural crest stem cells.

In an embodiment, the initial neural crest stem cells may be neural crest stem cells that correspond to a neural plate border-stage of an embryo neurulation stage.

In an embodiment, the initial neural crest stem cells may be neural crest stem cells expressing the PAX7 gene and a protein thereof.

In an embodiment, the initial neural crest stem cells may exist in the neural plate.

In an embodiment, the initial neural crest stem cells may be cells positive to PAX7, MSX1, AP2alpha, p75 NGFR, or HNK1, and more preferably, cells positive to p75 NGFR or HNK1.

In an embodiment, the agent for measuring the expression level of a gene may be an agent for measuring an expression level of mRNA of the gene or an expression level of a protein of the gene.

In an embodiment, the agent for measuring the expression level of mRNA of the gene may include a nucleic acid sequence of a marker, a nucleic acid sequence complementary to the nucleic acid sequence, a primer pair that specifically recognizes a fragment of the nucleic acid sequence and the nucleic acid sequence complementary to the nucleic acid sequence, a probe, or both a primer pair and a probe, and the agent may be measured by a method selected from the group consisting of polymerase chain reaction (PCR), real-time PCR (qPCR), reverse transcription PCR (RT-PCR), competitive RT-PCR, nuclease protection assay (e.g., RNase and S1 nuclease protection assay), in situ hybridization, nucleic acid microarray, northern blotting, or DNA chip.

In an embodiment, the agent for measuring the expression level of the protein of the marker may include an antibody, an antibody fragment, an aptamer, an avidity multimer, or a peptidomimetic, which specifically recognizes the full-length protein or a fragment thereof of the gene, and the agent may be measured by a method selected from the group consisting of western blotting, enzyme-linked immunosorbent assay (ELISA), radioimmunoassay (RIA), radioimmunodiffusion, immunoelectrophoresis, tissue immunostaining, immunoprecipitation assay, complement fixation assay, flow cytometry (FACS), mass spectrometry, or protein microarray.

In an aspect, the present disclosure relates to a composition for detecting neural crest stem cells having axial specification information, the composition including an agent for measuring the expression level of the ETS1 gene or the ZIC1 gene.

In an aspect, the present disclosure relates to a method of detecting neural crest stem cells having axial specification information, the method including measuring an expression level of the ETS1 gene or the ZIC1 gene in an isolated sample.

In an embodiment, the sample may be any material, a biological fluid, a tissue, or a cell derived from a subject.

In an embodiment, the method of detecting neural crest stem cells having axial specification information may further include determining cells as neural crest stem cells having axial specification information when the expression level of the ETS1 gene or the ZIC1 gene is increased or positive compared to a control group.

In an embodiment, the present disclosure relates to use of the agent for measuring the expression level of the ETS1 gene or the ZIC1 gene, for detecting neural crest stem cells having axial specification information.

In an embodiment, the present disclosure relates to use of the agent for measuring the expression level of the ETS1 gene or the ZIC1 gene, for preparing a composition for detecting neural crest stem cells having axial specification information.

In an embodiment, the present disclosure relates to use of the ETS1 gene or the ZIC1 gene, for detecting neural crest stem cells having axial specification information.

In an embodiment, the neural crest stem cells having axial specification information may be cranial neural crest stem cells, vagal neural crest stem cells, cardiac neural crest stem cells, trunk neural crest stem cells, or sacral neural crest stem cells, and more preferably, may be cranial neural crest stem cells.

In an embodiment, the agent for measuring the expression level of the gene may be an agent for measuring an expression level of mRNA of the gene or an expression level of a protein of the gene.

In an embodiment, the agent for measuring the expression level of mRNA of the gene may include a nucleic acid sequence of the marker, a nucleic acid sequence complementary to the nucleic acid sequence, a primer pair that specifically recognizes a fragment of the nucleic acid sequence and the nucleic acid sequence complementary to the nucleic acid sequence, a probe, or both a primer pair and a probe, and the agent may be measured by a method selected from the group consisting of PCR, qPCR, RT-PCR, competitive RT-PCR, nuclease protection assay (e.g., RNase and S1 nuclease protection assay), in situ hybridization, nucleic acid microarray, northern blotting, or DNA chip.

In one embodiment, the agent for measuring the expression level of the protein of the marker may include an antibody, an antibody fragment, an aptamer, an avidity multimer, or a peptidomimetic, which specifically recognizes the full-length protein or a fragment thereof of the gene, and the agent may be measured by a method selected from the group consisting of western blotting, ELISA, RIA, radioimmunodiffusion, immunoelectrophoresis, tissue immunostaining, immunoprecipitation assay, complement fixation assay, flow cytometry (FACS), mass spectrometry, or protein microarray.

The term "detection" or "measurement" used in the present specification may refer to quantification of a concentration of a detected or measured subject.

The term "primer" used in the present specification refers to a short nucleic acid sequence having a free 3-terminal hydroxyl group, which can form a base pair with a complementary template and functions as a starting point for copying a template strand. The primer may initiate DNA synthesis in the presence of a reagent for polymerization (i.e., a DNA polymerase or a reverse transcriptase) and four different nucleoside triphosphates with an appropriate buffer solution at an appropriate temperature.

In the present disclosure, the term "probe" refers to a fragment of nucleic acid, such as RNA or DNA, as short as a few bases or as long as several hundred bases, which can bind specifically to mRNA, and a probe is labeled to determine the presence or absence of specific mRNA. The probe may be designed in the form of an oligonucleotide probe, a single-stranded DNA probe, a doublestranded DNA probe, an RNA probe, or the like. In the present disclosure, hybridization is performed using a probe complementary to the gene, and the degree of expression of the gene may be diagnosed by hybridization. The selection of an appropriate probe and hybridization conditions may be modified based on those known in the art, and are not specifically limited in the present disclosure.

Primers or probes of the present disclosure may be chemically synthesized by using a phosphoramidite solid support method or other well-known methods. Such nucleic acid sequences may also be modified by using many means known in the art. Non-limiting examples of modifications may include methylation, encapsulation, substitution with one or more homologues of natural nucleotides, and modification between nucleotides, such as modification to uncharged linkages (e.g., methyl phosphonate, phosphotriester, phosphoramidate, carbamate, or the like) or to charged linkages (e.g., phosphorothioate, phosphorodithioate, or the like).

In the present disclosure, suitable conditions for hybridizing a probe with a cDNA molecule may be determined through a series of optimization procedures. These procedures are carried out as a series of steps by those skilled in the art to establish a protocol for use in the laboratory. For example, conditions such as temperature, concentration of components, hybridization and washing times, buffer components and pH thereof, and ionic strength depend on various factors including the length and GC content of probes and the target nucleotide sequence. Detailed conditions for hybridization are described in Joseph Sambrook, et al., Molecular Cloning, A Laboratory Manual, Cold Spring Harbor Laboratory Press, Cold Spring Harbor, N.Y.(2001); and M.L.M. Anderson, NucleicAcidHybridization, Springer-Verlag New York Inc. N.Y.(1999). For example, among the stringency conditions described above, high stringency conditions refer to hybridization at 65 °C in 0.5 M NaHPO4, 7 % sodium dodecyl sulfate (SDS), and 1 mM EDTA, and washing at 68 °C in 0.1 x standard saline citrate (SSC)/0.1 % SDS. Alternatively, high stringency conditions refer to washing at 48 °C in 6 x SSC/0.05 % sodium pyrophosphate. Low stringency conditions refer to, for example, washing at 42 °C in 0.2 x SSC/0.1 % SDS.

In the present disclosure, the term "antibody" is a term known in the art and refers to a specific protein molecule directed against an antigenic site. For the purpose of the present disclosure, the antibody refers to an antibody binding specifically to a protein expressed in the gene, and the antibody may be produced using a widely known method. Here, the antibody also includes a partial peptide that can be produced from the protein. The form of the antibody of the present disclosure is not particularly limited, and the antibody of the present disclosure includes a polyclonal antibody, a monoclonal antibody, or a part thereof as long as it has antigen binding properties, and any immunoglobulin antibody. Furthermore, the antibodies of the present disclosure may also include special antibodies such as humanized antibodies.

In an aspect, the present disclosure relates to a kit including the composition for detecting initial neural crest stem cells or neural crest stem cells of the present disclosure.

In an aspect, the present disclosure relates to a method of producing initial neural crest stem cells, the method including the steps of: culturing pluripotent stem cells; differentiating the cultured cells into initial neural crest stem cells; and isolating the initial neural crest stem cells.

In an embodiment, the initial neural crest stem cells may be neural crest stem cells that correspond to a neural plate border-stage of an embryo neurulation stage.

In an embodiment, the initial neural crest stem cells may be neural crest stem cells expressing the PAX7 gene and a protein thereof.

In an embodiment, the initial neural crest stem cells may exist in the neural plate.

In an embodiment, the initial neural crest stem cells may be cells positive to PAX7, MSX1, AP2alpha, p75 NGFR, or HNK1, and more preferably, cells positive to p75 NGFR or HNK1.

In an embodiment, the initial neural crest stem cells may be neural crest stem cells not forming axial information.

In an embodiment, the differentiation may be performed for 4 to 7 days.

In an aspect, the present disclosure relates to a method of producing neural crest stem cells, the method including the steps of: culturing pluripotent stem cells; performing first differentiation from the cultured cells into initial neural crest stem cells; isolating the initial neural crest stem cells; and performing second differentiation from the initial neural crest stem cells into neural crest stem cells having axial specification information.

In an embodiment, WNT, BMP, BMP4, SHH, FGF2, NOTCH or bFGF may be used for treatment during the second differentiation, and more preferably, fibroblast growth factor 2 (FGF2) may be used for treatment.

In an embodiment, bFGF, both FGF2 and BMP, or BMP4 may be treated together during the second differentiation.

In an embodiment, FGF2 may be treated at a concentration of 5 to 100 ng/ml during the secondary differentiation.

In an embodiment, the expression level of the ZIC1 gene during the secondary differentiation may decrease compared to before the secondary differentiation, and the expression ratio of the ETS1 gene/ZIC1 gene during the secondary differentiation may increase compared to before the secondary differentiation.

In an embodiment, the FGF2 may inhibit the ZIC1 gene expression during the secondary differentiation, and may promote the ETS1 protein expression through BMP4.

In an embodiment, the neural crest stem cell having axial specification information may be a cranial neural crest stem cell, a vagus neural crest stem cell, a cardiac neural crest stem cell, a trunk neural crest stem cell, or a sacral neural crest stem cell.

In an embodiment, the neural crest stem cell having axial specification information may be a cell expressing the SOX9 gene or the SOX10 gene, or a protein of the gene.

In an embodiment, the first differentiation may be performed for 4 to 7 days, and the second differentiation may be performed for 3 to 10 days.

In an embodiment, by precisely controlling and transmitting cell signals that directly affect neural crest stem cells while excluding the influence of non-neural crest stem cells by using the methods of the present disclosure, neural crest stem cells having axial specification information may be effectively and precisely produced. When the two-step differentiation method utilizes bFGF in an embodiment of the present disclosure, it was confirmed that the differentiation efficiency from SOX9 reporter hESCs into cranial neural crest stem cells was improved from about 30 % to about 80 % compared to the conventional methods (see FIGS. 8C and 8D).

By conventional technology of neural crest stem cell differentiation, cell signals, such as FGF, **WNT,** and the like, are activated to differentiate and obtain neural crest cells at each position in the body such that cranial neural crest cells, trunk neural crest cells, and the like are secured. However, this technology has a disadvantage of obtaining only one type of neural crest stem cell per batch. On the other hand, the present disclosure has developed a two-step neural crest stem cell differentiation technique to solve the problem that the conventional one-step neural crest stem cell differentiation method in the art has various limitations because this method is for differentiating and obtaining only one type of neural crest stem cells, thereby having the advantages of being able to simultaneously differentiate and obtain neural crest stem cells having various location information, such as cranial-, trunk-, cardiac-, and sacral- neural crest stem cells in a single batch.

In this regard, it is important to secure initial neural crest cells that have been differentiated from pluripotent stem cells but have not yet formed axial specification information, i.e., initial neural crest cells before the neurulation stage.

In this regard, in the present disclosure, it was confirmed initial neural crest cell labeling using p75 NGFR is possible through staining neural crest cells of a developing chick embryo, when human pluripotent stem cells are differentiated in a newly prepared differentiation medium (PIM), it was confirmed that the p75 NGFR protein is expressed before the expression of the SOX10 protein expressed by neural crest stem cells having migration ability with axial specification information. Accordingly, pluripotent stem cells were subjected to first differentiation for 5 to 7 days to be differentiated into initial neural crest cells whose axial specification information has not been determined were differentiated, and the cells were isolated using the p75 NGFR antibody. Then, during the second differentiation process of re-culturing these cells, the cells were differentiated into cranial neural crest stem cells when treated with bFGF and into trunk neural crest stem cells when treated with WNT. These cells were then verified through changes in the ZIC1 or ETS1 expression.

In addition, the differentiation methods for neural crest cells in the art may enable differentiation of cells into specific neural crest cells, but initial neural crest cells, which have not yet obtained axial specification information (those located in the neural tube during the neurulation stage and expressing the PAX7 protein), are limited in their ability to differentiate, acquire, or identify. In addition, since various cell types, including neural crest cells and non-neural crest cells, are mixed during a cell differentiation process, the exact mechanisms of signaling proteins such as bFGF and WNT are not understood.

On the other hand, since the present disclosure enables isolation of pure initial neural crest cells, it is possible to deliver signaling proteins, such as bFGF and WNT, to these cells as a target for more efficient differentiation and acquisition of axially specified neural crest stem cells. Furthermore, the roles of signaling proteins, such as BMP and SHH, which are known to play a role in axial specification of neural crest stem cells, are also examined to determine whether the signaling proteins act directly on neural crest cells or indirectly on non-neural crest stem cells present in a mixture and consequently affect neural crest cells.

As a result, the present disclosure confirmed through the methods that conditions, such as inhibitory effect of bFGF on ZIC1, inhibitory effect of bFGF on WNT function, and promotion of ETS1 by BMP4, for pure initial neural crest stem cells promote cranial-axial-specification of initial neural crest stem cells, and conditions, such as ZiC1 promotion by WNT and bFGF function inhibition by SHH, promote trunk-axial-specification of initial neural crest stem cells.

The present disclosure will be described in more detail with reference to Examples below. However, Examples below are intended to specify the present disclosure and are not intended to limit the present disclosure.

### Example 1. Screening of markers for initial neural crest stem cells (NCSCs)

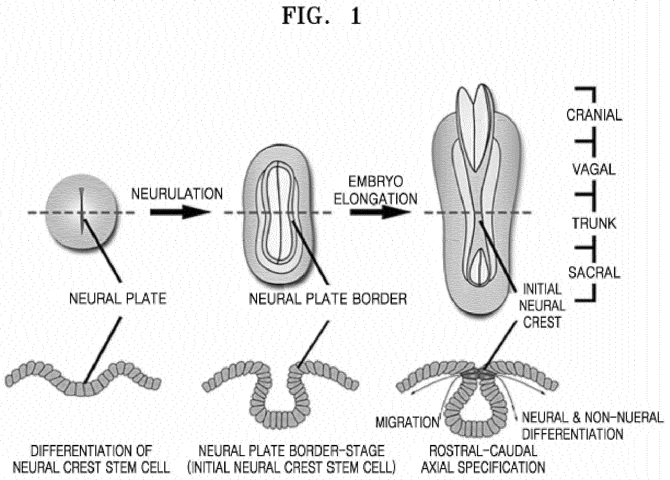

To screen for cell surface markers specific to initial NCSCs (prior to specification to cranial/vagal/trunk/sacral NCSCs) that have not yet formed human axial information as being in the neurulation stage of the human embryonic development (FIG. 1), the expression of p75 NGFR was confirmed after tissue clearing in the dorsal regional initial neural crest of developing chick embryos by immunofluorescence staining, and the expression of SOX10 and p75 NGFR was confirmed over the differentiation time in a differentiation process of human pluripotent stem cells into NCSCs.

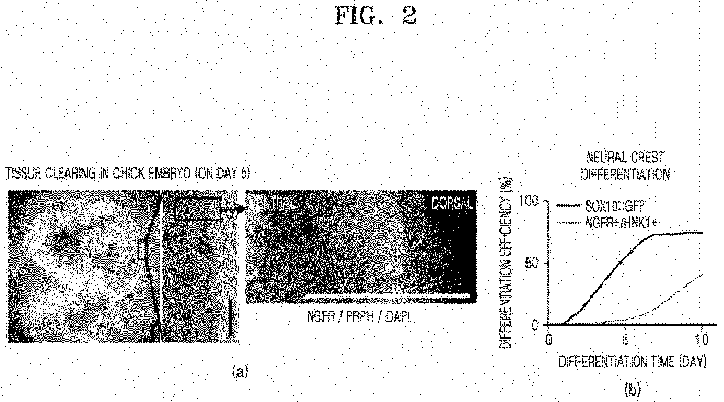

As a result, it was confirmed that the expression of NGFR/HNK precedes the expression of conventional SOX10, which labels migrating NCSCs, during the differentiation process of human pluripotent stem cells, and that pre-migrating initial NCSCs can be accordingly isolated by using the p75 NGFR antibody or the HNK1 antibody (FIG. 2).

### Example 2. First differentiation into initial NCSCs at neural plate border-stage

Human pluripotent stem cell (hESC) clones were made into single cells by using an appropriate solution such as Accutase (Innovative Cell Technologies) or Versene (Thermo Fisher), and 100,000 cells per well of a 24-well plate were seeded onto a Geltrex ECM-coated plate. When the density of hESCs reached 70 %, the maintenance medium of the hESCs was replaced with a differentiation medium, PIM medium (DMEM/F-12; Thermo Fishser, supplemented with 0.5 % KSR (Thermo Fishser), 2 % B-27 supplement (Thermo Fishser), and 1 % Glutamax (Thermo Fishser), pH 7.5) containing 3uM CHIR99021 (GSK-3 inhibitor, WNT activator) (Day 0). Then, the medium was replaced with PIM medium containing 3uM CHIR99021 on Days 2 and 4 and added every other day to produce initial NCSCs that correspond to the embryo neurulation stage and have not yet formed axial information in the body (Table 1).

**[Table 1]**

| DAY | Medium composition |
|---|---|
| Day 0 | PIM + CHIR99021 (3 uM) |
| Day 1 | PIM + CHIR99021 (3 uM) |
| Day 2 | PIM + CHIR99021 (3 uM) |
| Day 3 | PIM + CHIR99021 (3 uM) |
| Day 4 | PIM + CHIR99021 (3 uM) |

### Example 3. Isolation and identification of initial NCSCs at neural plate-border stage

### 3-1. Isolation of initial NCSCs

To isolate initial NCSCs, i.e., NCSCs at the neural plate border-stage, cells were collected on Day 5 or Day 7 of the differentiation by using Accutase, and the cell pellets were washed with PBS and re-suspended in FACS buffer. Multicellular clusters were removed by filtration performed twice using 35 um strainer snap-cap round bottom tubes, and then, initial NCSCs at the neural plate border-stage were isolated by a FACS sorter (SONY SH-800) by labeling with antibodies against NCSC surface antigens p75 NGFR or HNK1.

### 3-2. Identification of initial NCSCs

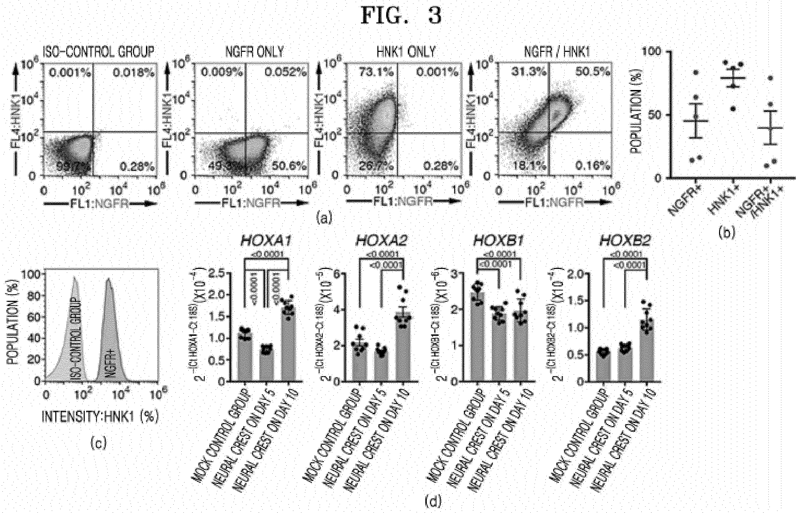

As a result of FACS analysis confirming the cells differentiated from pluripotent stem cells for 5 days, some of the cells appeared to be initial NCSCs expressing NGFR and/or HNK1, and cells expressing p75 NGFR were found to co-express HNK1 in many cases, with the average proportion of the p75 NGFR+/HNK1+ cells being 50 % (FIG. 3). In addition, the HOX gene expression pattern showed that initial NCSCs on Day 5 of the differentiation have not yet achieved cranial specification (FIG. 3).

Accordingly, it was confirmed that isolation of p75 NGFR positive cells alone is sufficient to isolate initial NCSCs.

### Example 4. Secondary differentiation into NCSCs having axial specification information

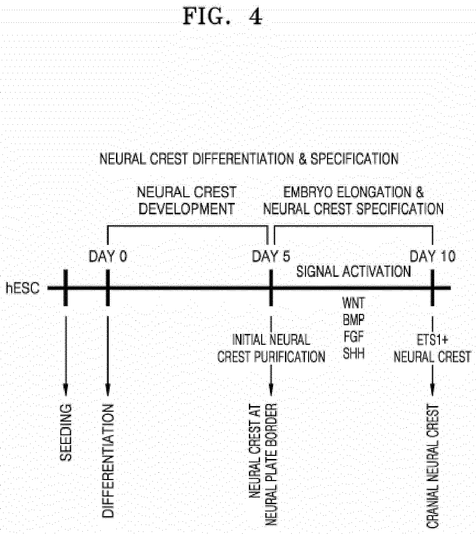

The NCSCs at the neural plate border-stage (cells on Day 5 of the differentiation) expressing p75 NGFR or HNK1 isolated by FACS in Example 3-1 were re-seeded at 300,000 cells per well in a 24-well plate coated with Geltrex, Matrigel, or Laminin+Fibronectin. After 24 hours, the PIM medium added on Day 5 for stabilization was gradually changed to a neurobasal medium containing 2 % B-27 supplement, 1 % N2 supplement, and 1 % Glutamax together with fibroblast growth factor 2 (FGF2) (Table 2). As an axial specification stimulating factor, FGF2 was added to the culture medium on days 6 to 10 of differentiation, and the proliferated neural crest stem cells were purified by FACS using an antibody against the neural crest stem cell surface antigen p75 NGFR on days 11 to 14 of differentiation (FIG. 4).

**[Table 2]**

| DAY | Medium Composition |
|---|---|
| Day 6 | 75 % PIM + 25 % neurobasal medium (NB) + FGF2 (20 ng/ml) |
| Day 8 | 50 % PIM + 50 % NB + FGF2 (20 ng/ml) |
| Day 10 | 25 % PIM + 75 % NB + FGF2 (20 ng/ml) |

### Example 5. Gene expression analysis of initial NCSCs

The expression of NCSC marker genes NGFR, HNK1, PAX7, MSX1, and AP2alpha, as well as the expression of NCSC differentiation promoter gene ZIC1 and cranial NCSC marker ETS1, in the initial NCSCs at the neural plate border-stage isolated by using the p75 NGFR antibody on Day 5 of the differentiation were analyzed by FACS, and the expression of PAX7 expressed at the neural plate border was confirmed by immunofluorescence analysis at the protein level.

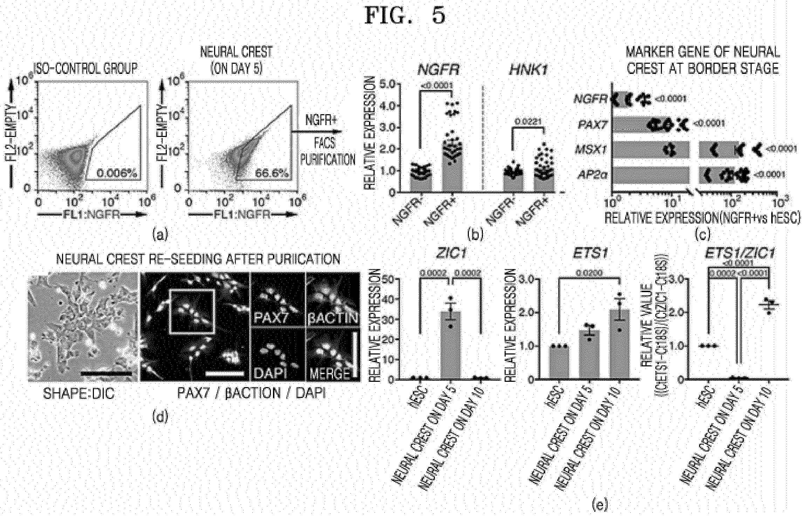

The results showed that the isolated initial NCSCs expressed NGFR, HNK1, PAX7, MSX1, and AP2alpha (FIGS. 5A to 5D). In addition, the initial NCSCs at the neural plate border-stage isolated on Day 5 of the differentiation were found to express significantly more ZIC1 compared to pre-differentiated cells and the NCSCs isolated on Day 10 of the differentiation, and the expression of ETS1 was found to increase as the differentiation progressed (FIG. 5E). Since the continuous expression of ZIC1 after ontogenetic differentiation is a specification factor for trunk NCSCs rather than cranial NCSCs, thus confirming that specification for cranial NCSCs occurred between Days 6 and 10 of the differentiation.

### Example 6. Analysis of gene expression changes in NCSCs undergoing axial specification

### 6-1. Gene expression analysis of NCSCs that have undergone second differentiation without axial specification stimulating factor

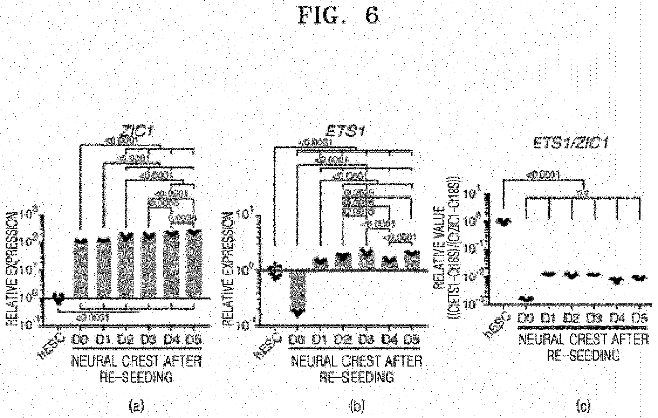

The initial NCSCs (expressing p75 NGFR or HNK1) on DAy 5 of the differentiation in Example 3-1 were isolated, or cultured as unisolated mixed cells for a second time for 5 days without addition of the axial specification stimulating factor FGF2. As a result of confirming the expression of the ZIC1 gene and ETS1 gene, it was found that the expression of the ZIC1 gene was not reduced, and the expression of the ETS1 gene was increased with the additional days of the differentiation days (FIGS. 6A and 6B). In addition, it was confirmed that the expression ratio (ETS1/ZIC1) of the ETS1 gene and the ZIC1 gene , which is indicative of the specification to cranial NCSCs, was maintained without being increased (FIG. 6C).

Accordingly, it was inferred that, when the initial NCSCs exist together with non-NCSCs during a differentiation process, the initial NCSCs can become committed to cranial NCSCs through cell-to-cell interactions, but the initial NCSCs alone fail to become committed to cranial NCSCs, and the main reason for this is the lack of reduction in the expression of the ZIC1 gene.

### 6-2. Gene expression analysis of NCSCs that have undergone second differentiation by adding axial specification stimulating factor

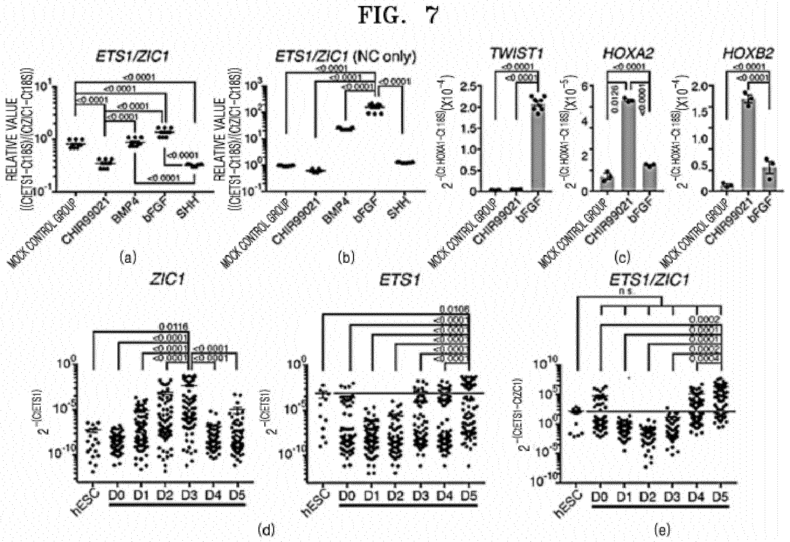

The expression of the ZIC1, ETS1, TWIST1, and HOX genes in the initial NCSCs (expressing p75 NGFR or HNK1) on Day 5 of the differentiation in Example 3-1 were isolated (NC only), or the axially specified NCSCs cultured as unisolated mixed cells for a second time for 5 days after treatment with an axial specification stimulating factors, such as WNT (WNT activator by treating with GSK-3 inhibitor CHIR99021), BMP, SHH or FGF2 were confirmed, and the results show that the treatment with FGF2 increased the expression ratio of ETS1/ZIC1 in both the isolated initial NCSC group and the unisolated mixed cell group (FIG. 7A), with a significant increase in the isolated initial NCSCs (FIG. 7B). Accordingly, it was confirmed that the role of the non-NCSCs is to activate FGF signaling of the initial NCSCs, inducing to cranial specification. In addition, the NCSCs treated with FGF2 showed increased expression of the TWIST1 gene, which is a marker gene of cranial NCSCs, and decreased expression of the HOX gene, which is known not to be expressed in cranial NCSCs (FIG. 7C). Furthermore, as a result of comparing the expression levels of the ZIC1 gene and ETS1 genes in single cells, it was shown that each single NCSC exhibited a significant decrease in the ZIC1 gene expression from Day 4 (FIGS. 7D and 7E).

Accordingly, it was confirmed that, when the isolated NCSCs exist alone, the ZIC1 gene does not reduce due to the lack of interactions with cells other than the NCSCs, whereas FGF2 can decrease the ZIC1 gene.

### Example 7. Mechanism analysis of axial specification retention of initial NCSCs by signal transduction factor.

The initial NCSCs were isolated on Day 5 of the differentiation, and then, on Days 5 or 10, the expression of the ETS1 protein was confirmed in the FGF2-treated NCSCs by immunochemical analysis, and the direct effects of various signaling factors on the initial NCSCs were confirmed by FACS analysis.

As a result, the FGF2 was shown to inhibit the ZIC1 gene expression and exhibit effects on promotion of the ETS1 protein expression through BMP4 (FIG. 8). When NCSCs were differentiated without FGF2 treatment using SOX9:reporter hESC, a known marker of cranial NCSCs, the differentiation efficiency into cranial NCSCs was approximately 25 %, and when treated with bFGF, the percentage of SOX9-expressing cells was 80 % or more (FIGS. 8C and 8D).

### Example 8. Confirmation of simultaneous generation of multiple axially specified NCSC populations

### 8-1. Confirmation of differentiation of multiple axially specified NCSCs

The NCSCs (those on Day 5 of the differentiation) at the neural plate border-stage obtained through the first differentiation in Example 3-1 were redistributed and each treated with bFGF or CHIR99021+ retinoic acid to be differentiated into cranial NCSCs or cardiac NCSCs. Thereafter, the mRNA expression of SOX9, TWIST1, and PRRX2, which are markers of cranial NCSCs, and the mRNA expression of cKIT, MEF2c, and MAFB, which are markers of cardiac NCSCs, were confirmed, and the results showed that the mRNA expression of SOX9, TWIST1, and PRRX2, which are markers of cranial NCSCs, was increased in the group treated with the bFGF (FIG. 9A), and that the mRNA expression of cKIT, MEF2c, and MAFB, which are markers of cardiac NCSCs, was increased in the group treated with CHIR99021+ retinoic acid to induce WNT activation (FIG. 10D).

### 8-2. Confirmation of differentiation potential of axially specified NCSCs into other cells

To confirm whether each of the NCSCs that have undergone the second differentiation in Example 8-1 differentiates into differentiation target cells at the corresponding human body location, the cranial NCSCs differentiated by treating the initial NCSCs with the bFGF in Example 8-1 differentiated into chondroblasts or osteoblasts, and then confirmed by staining with Alcian blue and Alizarin red to determine the expression of each relevant gene marker. In addition, the cardiac NCSCs differentiated by WNT activation with the initial NCSCs in Example 8-1 were differentiated into smooth muscle cells (SMCs), which were confirmed by immunofluorescence, and the expression of relevant marker genes was confirmed.

As a result, it was confirmed that the cranial neural crest stem cells were differentiated into the target cells, chondroblasts or osteoblasts (FIG. 9 (b) and (c)), and that the cardiac neural crest stem cells were differentiated into the target cells, SMCs (FIG. 10 (e) and (f)).

Accordingly, it was confirmed that multiple axially specified neural crest populations can be simultaneously generated within a single batch through the two-step differentiation method of the present disclosure.

## Claims

1. A composition for detecting initial neural crest stem cells, the composition comprising an agent for measuring an expression level of the p75 NGFR gene or the HNK1 gene.

2. The composition for detecting initial neural crest stem cells of claim 1, wherein the initial neural crest stem cells correspond to a neural plate border-stage of an embryo neurulation stage.

3. A composition for detecting neural crest stem cells having axial specification information, the composition comprising an agent for measuring an expression level of the ETS1 gene or the ZIC1 gene.

4. The composition for detecting neural crest stem cells of claim 3, wherein the neural crest stem cells having axial specification information are cranial neural crest stem cells, vagal neural crest stem cells, cardiac neural crest stem cells, trunk neural crest stem cells, or sacral neural crest stem cells.

5. A method of producing initial neural crest stem cells, the method comprising the steps of:
a) culturing pluripotent stem cells;
b) differentiating cultured cells into initial neural crest stem cells; and
c) isolating the initial neural crest stem cells.

6. The method of producing initial neural crest stem cells of claim 5, wherein the initial neural crest cells correspond to a neural plate border-stage of an embryo neurulation stage.

7. The method of producing initial neural crest stem cells of claim 5, wherein the initial neural crest stem cells do not form axial information.

8. The method of producing initial neural crest stem cells of any one of claims 5 to 8, wherein the initial neural crest stem cells are cells positive to PAX7, MSX1, AP2alpha, p75 NGFR, or HNK1.

9. A method of producing neural crest stem cells, the method comprising the steps of:
a) culturing pluripotent stem cells;
b) performing first differentiation from cultured cells into initial neural crest stem cells;
c) isolating the initial neural crest stem cells; and
d) performing second differentiation from the initial neural crest stem cells into neural crest stem cells having axial specification information.

10. The method of producing neural crest stem cells of claim 9, wherein the initial neural crest stem cells are cells positive to PAX7, MSX1, AP2alpha, p75 NGFR, or HNK1.

11. The method of producing neural crest stem cells of claim 9, wherein WNT, BMP, BMP4, SHH, fibroblast growth factor 2 (FGF2), NOTCH, or bFGF is treated during the second differentiation.

12. The method of producing neural crest stem cells of claim 9, wherein an expression level of the ZIC1 gene during the second differentiation is reduced compared to before the second differentiation.

13. The method of producing neural crest stem cells of claim 9, wherein an expression ratio of the ETS1 gene/ZIC1 gene during the second differentiation is increased compared to before the second differentiation.

14. The method of producing neural crest stem cells of any one of claims 9 to 13, wherein the neural crest stem cells having axial specification information are cranial neural crest stem cells, vagus neural crest stem cells, cardiac neural crest stem cells, trunk neural crest stem cells, or sacral neural crest stem cells.
